# FASCICULE DE BREVET EUROPEEN

(11) **EP 0 577 904 B1**
(45) Date de publication et mention de la délivrance du brevet: **14.05.1997**
(21) Numéro de dépôt: 92810516.2
(22) Date de dépôt: 06.07.1992
(51) Int. Cl.: C12N 1/20, A23K 1/00, A23C 9/127

(54) **Bactérie lactique**
Milchbakterien
Lactic bacteria

(43) Date de publication de la demande: 12.01.1994
(60) Demande divisionnaire: 96202911.2
(73) Titulaire: SOCIETE DES PRODUITS NESTLE S.A., 1800 Vevey (CH)
(72) Inventeur: Brassart, Dominique, CH-1030 Bussigny (CH); Donnet, Anne, CH-1806 Saint-Legier (CH); Link, Harriet, CH-1800 Vevey (CH); Mignot, Olivier, CH-1807 Blonay (CH); Neeser, Jean -Richard, CH-1010 Lausanne (CH); Rochat, Florence, CH-1820 Montreux (CH); Schiffrin, Eduardo, CH-1023 Crissier (CH); Servin, Alain, UFR de Sciences Pharmaceutiques, F-92296 Chatenay-Malabry (FR)
(74) Mandataire: Wavre, Claude-Alain

(56) Documents cités:
- EP-A- 0 199 535
- IMMUNOLOGY, vol. 63, no. 1, 1988, Oxford (GB); G. PERDIGON et al., pp. 17-23
- MEDLINE DATABASE, Bethesda, MD (US); F. BALLI et al., AN 92253433
- BIOLOGICAL ABSTRACTS, vol. 79, Philadelphia, PA (US); R. DUCLUZEAU et al., AN 252134
- MEDICINA, vol. 46, no. 6, 1986, Buenos Aires (AR); G. PERDIGON et al., pp. 751-754
- PATENT ABSTRACTS OF JAPAN, vol. 13, no. 579(C-668), 20 décembre 1989

## Description

La présente invention a pour objet une culture biologiquement pure d'une souche de bactérie lactique, une composition contenant cette souche et une utilisation de cette souche.

EP 199535 (Gorbach et Goldin) propose une souche bactérienne identifiée tout d'abord comme étant un Lactobacillus (L.) acidophilus, puis comme se rapprochant le plus de L.casei subs. ramnosus (cf M.Silva et al. dans Antimicrobial Agents and Chemotherapy, 31, No 8, 1231-1233, 1987), qui présente une bonne adhésion aux cellules de la muqueuse de l'intestin grêle et qui se prête à des utilisations thérapeutiques. Cette souche, baptisée souche GG et déposée à l'ATCC (American Type Culture Collection) sous le No 53103, peut être utilisée en combinaison avec un support pharmaceutiquement acceptable, en particulier dans des produits alimentaires, notamment dans des produits laitiers acidifiés du type yogourt, par exemple.

D'autres souches du même type ont été utilisées de longue date dans des produits et dans un but analogues. Il existe cependant un besoin de souches particulièrement performantes de ce type qui soient clairement identifiées, qui présentent des vertus indiscutables et qui viennent enrichir la palette des souches disponibles.

La présente invention a pour but de satisfaire ce besoin.

A cet effet, selon la présente invention, on propose une culture biologiquement pure d'une souche de bactérie lactique sélectionnée pour ses facultés d'implantation dans une flore intestinale, d'adhésion à des cellules intestinales, d'exclusion compétitive de bactéries pathogènes sur des cellules intestinales, d'immunomodulation et/ou de réduction d'activité enzymatique fécale.

Une telle souche est tout particulièrement destinée à être administrée à l'homme ou à des animaux dans un but thérapeutique ou prophylactique au niveau du système gastro-intestinal, notamment dans un but antidiarrhéique.

La souche peut être administrée sous forme de culture biologiquement pure, telle quelle, après congélation et/ou lyophilisation, par exemple. Une telle culture peut comprendre de 10⁸-10¹⁰ germes viables (cfu, de l'expression technique anglaise "colony forming units") par g pour la forme liquide ou congelée, et de 10⁹-10¹¹ cfu/g pour la forme lyophilisée, par exemple.

La souche peut également être administrée sous forme d'une composition comprenant une telle culture et un support ingérable, en particulier un support pharmaceutiquement acceptable ou un produit alimentaire tel qu'un lait acidifié, notamment un yogourt ou une formule lactée en poudre, par exemple.

Dans un premier mode de réalisation préféré de la présente invention, on propose une culture d'une souche de bactérie lactique sélectionnée pour sa faculté de s'implanter dans le tube digestif de souris ou de rats à flore intestinale humaine.

Dans un deuxième mode de réalisation préféré de la présente invention, on propose une culture d'une souche de bactérie lactique sélectionnée pour sa faculté d'exclusion compétitive, sur des cellules intestinales, de bactéries pathogènes responsables de diarrhées.

Parmi diverses souches de bactéries ainsi sélectionnées à partir notamment de laits acidifiés, en particulier de yogourts du commerce, ou à partir de cultures du commerce destinées à la préparation de tels laits, ou à partir de fèces d'enfants, par exemple, quatre ont été déposées, à titre d'exemple, selon le traité de Budapest, le 30.06.92, à la Collection Nationale de Cultures de Microorganismes (CNCM), Institut Pasteur, 28 rue du Dr Roux, 75724 Paris Cedex 15, France, où elles ont reçu chacune le No CNCM respectif indiqué ci-après entre parenthèses, à savoir une souche de Lactobacillus acidophilus (CNCM I-1225), une souche de Bifidobacterium breve (CNCM I-1226), une souche de Bifidobacterium infantis (CNCM I-1227) et une souche de Bifidobacterium longum (CNCM I-1228).

Des détails concernant la morphologie et les propriétés générales de ces souches sont donnés ci-après:

### L.acidophilus CNCM I-1225

### Morphologie:

- Microorganisme Gram-positif, non motile, ne formant pas de spores.
- Bâtonnets isolés assez courts et trapus

### Métabolisme:

- Microorganisme microaérophile, avec métabolisme homofermentaire donnant lieu à la production d'acide lactique L(+) et D(-).
- Autres caractéristiques : Catalase (-), production de CO₂ (-), hydrolyse de l'arginine (-).

### Fermentation des sucres:

amygdaline (+), arabinose (-), cellobiose (+), esculine (+), fructose (+), galactose (-), glucose (+), lactose (+), maltose (+/-), mannitol (-), mannose (+), melibiose (-), raffinose (+), ribose (-), salicine (+), sucrose (+), trehalose (+).

### Implantation dans une flore intestinale

### Souris gnotoxéniques

Deux groupes de souris axéniques (souris sans flore intestinale) sont associées chacune avec la flore humaine d'un donneur différent (souris gnotoxéniques). Après plusieurs jours d'installation, les flores intestinales des souris sont tout à fait comparables à celles des donneurs humains, d'un point de vue fonctionnel, qualitatif et quantitatif.

Dans le cadre de la présente invention, de nombreuses souches sont testées pour leur aptitude à coloniser le tube digestif de ces souris à flore humaine, autrement dit pour leur faculté d'implantation dans cette flore intestinale.

On constate que la plupart des souches ne sont pas capables de coloniser ces animaux, même après plusieurs inoculations successives, mais que la souche L.acidophilus CNCM I-1225, par exemple, est capable de se multiplier et de s'implanter dans le tube digestif, autrement dit dans la flore intestinale des souris des deux groupes, et ceci même après une seule inoculation.

Cette colonisation ou implantation permet à la souche d'être présente dans les fèces à raison de plus de 10⁶cfu/g. Cette teneur des fèces en germes viables de la souche peut être considérée comme nécessaire et/ou suffisante pour que le métabolisme de la souche puisse modifier celui de l'hôte.

On constate en outre que cette implantation persiste tant que l'environnement des animaux n'est pas perturbé.

### Rats gnotoxéniques

Des rats axéniques sont associés (rats gnotoxéniques) avec une souche (Bacteroïdes thetaiotaomicron FI 1, collection particulière du Centre de Recherche Nestec SA, CH-1000 Lausanne, Suisse) isolée d'une flore intestinale humaine d'un donneur bien portant destinée, voir plus loin, à simuler la production d'enzymes d'une flore fécale complète. Cette association entraîne une colonisation abondante de l'intestin de ces rats et permet à cette bactérie de se retrouver dans les fèces à raison de environ 10⁸ cfu/g.

Un essai d'implantation de la souche de L.acidophilus CNCM I-1225, par exemple, dans cette flore résulte en une bonne co-colonisation qui permet à cette souche d'être également présente dans les fèces à raison de environ 10⁸ cfu/g.

### Volontaires humains

A titre de comparaison, on détermine combien de germes viables de L.bulgaricus se retrouvent dans les fèces de volontaires humains bien portants qui consomment du yogourt traditionnel, préparé par fermentation d'un lait de vache avec une culture de L.bulgaricus et de S.thermophilus du commerce.

Ces volontaires ne consomment aucun produit laitier fermenté durant trois périodes consécutives de trois semaines chacune, exception faite du yogourt qu'ils consomment durant la deuxième période de trois semaines.

Durant les trois semaines où ils consomment du yogourt, ils le font de manière à ingérer environ 10¹⁰ L.bulgaricus par jour, ce qui correspond à environ 3 yogourts de 120 g par jour. Durant la période de consommation des yogourts, on retrouve environ 10⁵ cfu de L.bulgaricus par g de fèces des volontaires.

Dans le cadre de la présente invention, on réalise un essai selon le même scénario que ci-dessus, mais avec du yogourt préparé par fermentation d'un lait avec une culture de S.thermophilus et de B.bifidus du commerce supplémentée avec la souche L.acidophilus CNCM I-1225, par exemple, en concentration du même ordre.

On détermine le nombre total de germes viables de Lactobacilles dans les fèces des volontaires avant, pendant et après la période de consommation du yogourt. On trouve des valeurs de 10⁵-10⁶ cfu/g avant, plus de 10⁷ cfu/g pendant et 10⁶ cfu/g après.

On observe donc une augmentation du nombre total de Lactobacilles retrouvés dans les fèces pendant la période de consommation du yogourt. La souche CNCM I-1225 est retrouvée en quantité importante et sous forme viable chez les volontaires. Par contre, elle est éliminée en quelques jours après l'arrêt de la consommation du yogourt.

### Réduction d'activité enzymatique fécale

### Rats gnotoxéniques

Dans le cadre des essais réalisés avec les rats gnotobiotiques ci-dessus, on détermine l'activité azoréductase et nitroréductase fécale. Les enzymes azoréductase et nitroréductase sont en effet impliquées dans la production de substances carcinogènes. Une concentration élevée de ces enzymes est liée à une augmentation de risque du cancer du colon.

On constate que l'activité enzymatique fécale des rats gnotobiotiques à Bactéroïdes se monte à 2,5 µg/h/mg de protéine pour l'azoréductase et à 4,2 µg/h/mg de protéine pour la nitroréductase, alors que, pour les rats gnotobiotiques à Bactéroïdes dans la flore desquelles la souche CNCM I-1225, par exemple, a été implantée, cette activité enzymatique se monte à 1,8 µg/h/mg de protéine pour l'azoréductase et à 3,5 µg/h/mg de protéine pour la nitroréductase.

On constate par ailleurs que des rats gnotobiotiques à flore intestinale formée exclusivement de la souche CNCM I-1225 ne présentent aucune activité azoréductase et nitroréductase fécale.

En d'autres termes, on constate que la présence de la souche CNCM I-1225 dans la flore de rats gnotobiotiques induit une diminution de certaines productions enzymatiques indésirables chez ces animaux, autrement dit des modifications bénéfiques dans le métabolisme de l'hôte.

### Volontaires humains

Dans le cadre des essais réalisés avec les volontaires humains ci-dessus, on détermine l'activité enzymatique nitroréductase fécale. On détermine cette activité durant les derniers jours qui précèdent la période de consommation du yogourt préparé avec la souche CNCM I-1225, par exemple, tout au long de cette période et dans les premiers jours qui suivent.

On constate ainsi que cette activité passe de 8,2 à 4,9 µg/h/mg de protéine durant la période de consommation du yogourt, qu'elle reste à ce niveau durant environ une semaine après cette période et qu'elle remonte progressivement ensuite.

### Immunomodulation

### Volontaires humains (pouvoir phagocytaire des leucocytes)

Des volontaires humains s'abstiennent de toute consommation de produits laitiers fermentés, à l'exception des produits consommés selon le programme suivant: lait durant trois semaines, yogourt préparé par fermentation d'un lait avec une culture mixte de S.thermophilus du commerce et de L.acidophilus CNCM I-1225, par exemple, durant les trois semaines suivantes, puis lait durant six semaines.

On détermine le pouvoir phagocytaire des leucocytes dans le sang périphérique des volontaires, au début et à la fin de chacune de ces périodes.

Cette détermination consiste à extraire les leucocytes du sang et à les mettre en présence de bactéries fluorescentes. On mesure la lumière fluorescente émise par les leucocytes qui ont phagocyté des bactéries fluorescentes par analyse cytométrique en flux (avec un appareil commercialisé sous le nom de Facscan). On en déduit le pourcentage des leucocytes qui ont une activité phagocytaire, pourcentage qui représente ledit pouvoir phagocytaire.

On observe un pouvoir phagocytaire des leucocytes du sang périphérique de 36,5% au début de la première période de consommation de lait, de 32,7% à la fin de cette période et donc au début de la période de consommation de yogourt, de 51,8% à la fin de cette période de consommation de yogourt et de 51,4% six semaines après, donc à la fin de la deuxième et dernière période de consommation de lait. La probabilité que l'on fasse erreur (valeur p) en estimant que cette augmentation du pouvoir phagocytaire des leucocytes est significative est inférieure à 0,1% .

### Volontaires humains (réponse à un vaccin)

16 volontaires humains bien portants (groupe test) suivent le programme d'alimentation suivant: durant deux semaines (semaines 1 et 2) diète normale dont est exclu tout produit fermenté, durant les trois semaines suivantes (semaines 3, 4 et 5) diète assortie de la consommation de trois yogourts de 125 ml par jour, ces yogourts ayant été préparés par fermentation d'un lait avec une culture de S.thermophilus et de Bifidobacterium bifidus du commerce à laquelle on a ajouté, à titre d'exemple, la souche de L.acidophilus CNCM I-1225 qui est présente dans ce yogourt à raison de 10⁷-10⁸ cfu/ml, et durant encore deux semaines (semaines 6 et 7) diète normale dont est exclu tout produit fermenté.

14 volontaires humains bien portants (groupe témoin) suivent simultanément un programme d'alimentation consistant en une diète normale dont est exclu tout produit fermenté.

Un vaccin oral vivotif (Salmonella typhi Ty21a) commercialisé par la maison Berna SA est administré aux volontaires des deux groupes conformément aux instructions du fabriquant aux jours 1, 3 et 5 de la semaine 4.

Des prises de sang sont effectuées sur tous les volontaires 3 jours avant le début de la semaine 3, ainsi que 1 jour et 10 jours après la fin de la semaine 5.

Une détermination de la concentration des IgA spécifiques de la réponse immunitaire aux lipopolysaccharides antigènes (LPS) de Salmonella typhi est réalisée par la méthode ELISA.

On constate que l'augmentation de la concentration des IgA spécifiques observée quinze jours après la vaccination, par rapport à la concentration observée neuf jours avant la vaccination, est significative dans les deux groupes (valeur p < 0,001).

Cependant, si l'on considère des plages de facteurs d'augmentation < 2; > 2 et < 3; > 3 et < 4; > 4, on observe des répartitions respectives dans ces plages de 1, 6, 3 et 6 volontaires pour le groupe test contre 8, 3, 0 et 3 volontaires pour le groupe témoin. En d'autres termes, les facteurs d'augmentation sont significativement plus élevés dans le groupe test que dans le groupe témoin (valeur p = 0,04).

### Adhésion à des cellules intestinales

Dans le cadre de la présente invention, on étudie l'adhésion de différentes souches de bactéries lactiques à des cellules intestinales, notamment à des cellules épithéliales intestinales humaines Caco-2 (M.Pinto et al., Biol. Cell. 47, 323, 1983) et à des cellules intestinales humaines sécrétrices de mucus HT29-MTX (Lesuffleur et al., Cancer Res. 50, 6334-6343) en culture monocouche in vitro.

Pour ce faire, on cultive les cellules dans des flacons en plastique de 25 cm² (Corning) pour l'entretien des lignées cellulaires et sur des lames de verre (22x22mm) dégraissées, stérilisées et placées dans des boîtes à six puits (Corning), pour les tests d'adhésion.

Pour cultiver les cellules Caco-2 et HT29-MTX, il faut changer quotidiennement le milieu à partir du deuxième jour suivant le réensemencement. On prépare le milieu de culture à partir de poudre de milieu essentiel minimum Eagle modifié par Dulbecco (DMEM).

Les bactéries lactiques sont cultivées en anaérobiose sur milieu MRS à partir d'un stock congelé. Les bactéries sont utilisées à partir du deuxième repiquage.

Un milieu mixte d'incubation sur les cellules est préparé en mélangeant 50% d'un milieu DMEM sans antibiotique et 50% du milieu MRS dans lequel les bactéries ont poussé, ce milieu contenant 10⁸ lactobacilles ou bifidobactéries (cf Chauvière G. et al., FEMS Microbiol.Lett.91, 213-218, 1992).

Pour réaliser l'adhésion, on dispose le milieu mixte contenant les bactéries sur les cellules intestinales et l'on incube une heure en aérobiose. On effectue cinq lavages par vingt agitations circulaires des boîtes à puits, de façon à permettre une bonne élimination des bactéries non adhérentes. Les tapis cellulaires sont ensuite fixés dans des bains successifs de méthanol, 10 min à 70%, 10 min à 95% et 15 min à 100%, et colorés à la coloration de Gram ou de Giemsa. Un niveau d'adhésion est déterminé par comptage microscopique des bactéries adhérentes.

Parmi les nombreuses souches essayées, la souche CNCM I-1225, présente un bon niveau d'adhésion à des cellules intestinales tel que déterminé par ces essais d'adhésion sur la lignée de cellules Caco-2.

C'est ainsi que la souche L.acidophilus CNCM I-1225 adhère aux cellules Caco-2 à raison de environ 150+23 cellules de bactérie pour 100 cellules Caco-2.

Des essais d'adhésion aux cellules HT29-MTX de la souche L.acidophilus CNCM I-1225, par exemple, donnent même des résultats encore plus spectaculaires.

On constate également, de manière surprenante, que l'adhésion de cette souche est due à un facteur qu'elle sécrète dans son propre milieu de culture (MRS ou lait, par exemple). En effet, si le processus d'incubation d'une heure sur Caco-2 décrit ci-dessus est réalisé avec la souche L.acidophilus CNCM I-1225 sans son milieu de culture bactérien, on observe une importante diminution de l'adhésion.

En outre, si l'on réalise ce processus d'incubation sur Caco-2 avec cette souche et son milieu de culture préalablement soumis à un traitement à la trypsine, on observe également une importante diminution de l'adhésion. Ceci semble prouver que le facteur d'adhésion sécrété par cette souche dans son milieu de culture est une protéine.

### Exclusion compétitive de bactéries pathogènes

Dans le cadre de la présente invention, on étudie les facultés, présentées par la souche CNCM I-1225, d'exclusion compétitive de bactéries pathogènes, notamment de bactéries pathogènes responsables de diarrhées, sur des cellules intestinales.

On étudie en particulier l'exclusion de certaines souches d'E.coli, saprophytes du tube digestif chez l'homme et l'animal, qui peuvent acquérir des caractères de virulence et devenir pathogènes, à savoir des E.coli entérotoxinogènes (ETEC), des E.coli entéroadhérents (DAEC) et des E.coli entéropathogènes (EPEC), ainsi que l'exclusion d'une souche de Salmonella typhi-murium.

Les souches utilisées pour cette étude sont:
- pour ETEC, la souche H10407 qui exprime CFA/1 (Collection du professeur Joly, Laboratoire de microbiologie, Faculté de médecine et de pharmacie, Université de Clermont-Ferrand 1, 63003 Clermont-Ferrand, France)
- pour DAEC, la souche C1845 (Collection of Dr S.Bilge, Department of Microbiology, School of Medicine, G 3111 Health Sciences Building, University of Washington, Seattle, Washington 98195, USA)
- pour EPEC, la souche JPN15 pMAR7 qui exprime EAF et eae (Collection of Prof. J.Kaper, Center for Vaccine Development, University of Maryland, School of Medicine, 10 South Pine Street, Baltimore, Maryland 21201, USA)
- pour Salmonella typhi-murium, la souche SL 1344 (Dr B.Stocker, Stanford University, School of Medicine, Department of Microbiology and Immunology, Sherman Sairchild Science Building, D 333 Stanford, California 94305-5402, USA)

Pour déterminer l'adhésion des bactéries sur les cellules Caco-2, on procède de la manière décrite ci-après:
En bref, on lave deux fois les monocouches de cellules Caco-2 avec un tampon phosphate salin (PBS). Les E.coli marquées au ¹⁴C ou les Salmonella marquées au ³⁵S sont mises en suspension dans le milieu de culture, à raison de 10⁸ cfu/ml, et 2ml de suspension sont ajoutés à chaque puits contenant une lame portant la culture cellulaire.

Pour E.coli, toutes les incubations sont conduites en présence de 1% de D-mannose. Pour déterminer un facteur ou taux d'exclusion, autrement dit quelle proportion de bactéries pathogènes sont empêchées d'adhérer aux cellules Caco-2 par des bactéries lactiques qui prennent leur place, on ajoute 1 ml de suspension contenant 10⁸ cfu/ml de souche pathogène marquée et 1 ml d'une suspension contenant soit 10⁸, soit 10⁹ cfu/ml de la souche de bactérie lactique testée, à chaque puits contenant une lame portant la culture cellulaire.

On incube les plaques à 37°C en atmosphère à 10% de CO₂ et 90% d'air durant 1 h. On lave les monocouches de cellules cinq fois au PBS stérile. Les bactéries adhérentes et les cellules intestinales sont dissoutes en solution de NaOH 0,2N. On évalue le nombre de bactéries adhérentes marquées par comptage à scintillation liquide.

Parmi différentes souches de bactéries lactiques ainsi testées quant à leurs facultés ou pouvoir d'exclusion compétitive de bactéries pathogènes, la souche CNCM I-1225, sélectionnée et déposée dans le cadre de la présente invention, a effectivement révélé des facultés remarquables présentées dans le tableau ci-après qui donne, en %, les taux d'exclusion réalisés par les souches testées aux dépens des différentes souches pathogènes utilisées dans ces essais.

| Souche (No CNCM) | Concentration (cfu/ml) | Taux d'exclusion compétitive (%), vis-à-vis de: | | | |
|---|---|---|---|---|---|
| | | ETEC | DAEC | EPEC | Salmonella |
| I-1225 | 10⁹ | 78 | 79 | 83 | 86 |
| | 10⁸ | 50 | 53 | 53 | 42 |
| I-1226 | 10⁹ | 80 | 68 | 83 | 88 |
| | 10⁸ | 55 | 53 | 55 | 41 |
| I-1228 | 10⁹ | 47 | 47 | | |
| | 10⁸ | 11 | | | |
| I-1227 | 10⁹ | 58 | 46 | | |
| | 10⁸ | 18 | | | |

## Revendications

1. Bactérie lactique ayant le numéro de dépôt CNCM I-1225.

2. Composition alimentaire ou pharmaceutique comprenant la bactérie lactique ayant le numéro de dépôt CNCM I-1225.

3. Composition selon la revendication 2, ladite composition étant un lait acidifié ou une formule lactée en poudre.

4. Utilisation la bactérie lactique ayant le numéro de dépôt CNCM 1-1225, dans la préparation d'un support administrable à l'homme ou à des animaux dans un but thérapeutique ou prophylactique au niveau du système gastro-intestinal.

## Patentansprüche

1. Milchsäurebakterie mit der Hinterlegungsnummer CNCM I-1225.

2. Nahrungsmittelzusammensetzung oder pharmazeutische Zusammensetzung, die eine Milchsäurebakterie mit der Hinterlegungsnummer CNCM I-1225 enthält.

3. Zusammensetzung nach Anspruch 2, wobei diese Zusammensetzung eine angesäuerte Milch oder eine Trockenmilchformulierung ist.

4. Verwendung der Milchsäurebakterie mit der Hinterlegungsnummer CNCM I-1225 in einem Präparat mit einem an den Menschen oder an Tiere verabreichbaren Träger mit einem therapeutischen oder prophylaktischen Ziel im Bereich des Gastro-Intestinal-Systems.

## Claims

1. Lactic bacterium having the registration number CNCM I-1225.

2. Food or pharmaceutical composition comprising the lactic bacterium having the registration number CNCM I-1225.

3. Composition according to claim 2, the said composition being an acidified milk or a powdered milk formula.

4. Use of the lactic bacterium having the registration number CNCM I-1225, in the preparation of a support which can be administered to man or to animals with therapeutic or prophylactic objectives in the gastro-intestinal system.
